(19) Europäisches Patentamt
European Patent Office
Office européen des brevets

(11) Publication number: **0 125 900**
**B1**

(12) **EUROPEAN PATENT SPECIFICATION**

(45) Date of publication of patent specification: **23.08.89**

(21) Application number: **84303195.6**

(22) Date of filing: **11.05.84**

(51) Int. Cl.$^4$: **A 61 K 31/195,**
A 61 K 31/405,
A 61 K 31/685, A 61 K 31/70
// (A61K31/195,
31:14),(A61K31/405,
31:14),(A61K31/70, 31:685,
31:405, 31:195, 31:14)

(54) **A pharmaceutical composition for use in treating neurological disease or aging.**

(30) Priority: **16.05.83 US 495202**

(43) Date of publication of application:
**21.11.84 Bulletin 84/47**

(45) Publication of the grant of the patent:
**23.08.89 Bulletin 89/34**

(84) Designated Contracting States:
**AT BE CH DE FR GB IT LI LU NL SE**

(56) References cited:
**EP-A-0 001 924**
**EP-A-0 005 057**
**EP-A-0 005 058**
**EP-A-0 005 333**
**EP-A-0 018 550**
**WO-A-80/02111**
**WO-A-80/02372**

**DICTIONNAIRE VIDAL, 1961, O.V.P., Paris (FR);**
**ROTE LISTE, 1965, Editio Cantor, Aulendorf (DE);**
**ROTE LISTE, 1961, Editio Cantor, Aulendorf (DE);**

(73) Proprietor: **MASSACHUSETTS INSTITUTE OF TECHNOLOGY**
**77 Massachusetts Avenue**
**Cambridge, MA 02139 (US)**

(72) Inventor: **Wurtman, Richard J.**
**193 Marlborough Street**
**Boston Massachusetts 02116 (US)**

(74) Representative: **Holdcroft, James Gerald, Dr. et al**
**Graham Watt & Co. Riverhead**
**Sevenoaks Kent TN13 2BN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 125 900 B1**

**Description**

The Government of the United States of America has rights in this invention pursuant to a grant by the National Institutes of Health of the United States.

This invention is concerned with potentiating the effect of neurotransmitters in the brain.

In normal aging, the brain loses neurons, including those that are dependent on the diet and blood stream for the precursors of their neurotransmitters, for example, acetylcholine-releasing or "cholinergic" neurons, which make acetylcholine from dietary lecithin or circulating choline; "catecholaminergic" neurons, that make dopamine, norepinephrine, or epinephrine from tyrosine; "serotoniergic" neurons, that synthesize serotonin from tryptophan; or "glycinergic" neurons, that can produce glycine from the amino acid threonine. Neuronal cell loss is specifically exacerbated in particualr neurological diseases, such as senility or Alzheimer's Disease in which cholinergic neurons are especially deficient, but catecholaminergic neurons may also be lost; or Parkinson's Disease, which especially affects dopaminergic neurons. Unfortunately, there is no presently available means to determine in a particular normal old person, or a person with a neurological disease, how many of which neuronal types have been lost. Moreover, a treatment that replaces one of the deficient neurotransmitters might be of limited utility if another transmitter were also deficient. Ideally, a treatment for this neuronal loss would provide the brain with agents that could increase the synthesis and release any of several transmitters, but which would have an effect only if each transmitter's release were deficient.

It is known that giving experimental animals choline enhances acetylcholine synthesis in rapidly-firing cholinergic neurons, and therefore is useful in treating disease states characterised by inadequate acetylcholine release, for example, Alzheimer's Disease, in which the surviving neurons presumably fire frequently, to make up for the missing ones, and also as a supplement to drugs which either act by releasing acetylcholine or which, as a side-effect, deplete neurons of acetylcholine. It is also known that giving tyrosine similarly enhances catecholamine release from rapidly-firing neurons, and that giving tryptophan or threonine enhances serotonin or glycine production in serotoninergic or glycinergic neurons, respectively. It is also known that the effectiveness of giving any of these amino acids can be potentiated by providing the amino acid in the proper ratio to carbohydrates which elicit insulin secretion, and which thereby lower plasma levels of other amino acids that compete with the desired one for uptake into the brain.

It is an object of the invention to potentiate the effect of neurotransmitter precursors when administered to a patient.

In order to obtain a synergistic effect when a combination of neurotransmitter precursors are administered to a patient, four conditions must exist, as exemplified by the interactions of tyrosine and choline. First, it is known that within a brain region such as corpus striatum, direct reciprocal synapses must exist between dopaminergic cells and e.g., cholinergic cells (in the case of tyrosine and choline). Second, it is known that cholinergic cells and dopaminergic cells both have the property of making and releasing more of their neurotransmitters when exposed to greater amounts of the precursor if the cells are firing frequently. Prior to this invention, it was not known that the necessary third and fourth conditions existed. The their condition is that acetylcholine release by the cholinergic cells must increase the firing of the dopaminergic cells, thereby making them tyrosine dependent. The fourth condition is that the loss of some dopaminergic neurons such as by aging or disease also causes the surviving dopaminergic neurons to fire frequently and thus become tyrosine-dependent.

The present inventor has found that the concomitant administration of (a) an amino acid which is a precursor to a neurotransmitter, i.e., tyrosine (or phenylalanine), tryptophan or threonine; and (b) choline or a choline precursor results in synergistically increased release of both thir corresponding neurotransmitters, i.e. respectively (a) dopamine serotonin or glycine; and (b) acetylcholine. The choline or choline precursor and amino acid are administered to a patient concomitantly. The coadministration of these compositions is particularly useful for patients affected by neurological disease including senility, Alzheimer's Disease or Parkinson's Disease, but also in normal older people, or people with obscure deficits in neurons releasing particular neurotransmitters.

The invention provides a pharmaceutical composition comprising (a) at least one amino acid selected from phenylalanine, tyrosine, threonine, and tryptophan; and (b) choline, a choline precursor or a mixture of choline and its precursor in amounts sufficient to cause a synergistic enhancement of neurotransmission; the composition being substantially free of other amino acids.

Rote Liste, page 97 (1961) discloses a composition mace up of, *inter alia*, choline, tryptophan and tyrosine; Dictoinnaire Vidal, page 1249 (1961) discloses a composition containing, *inter alia*, threonine and tryptophan plus choline citrate; Rote Liste page 525 (1965) discloses a composition containing, *inter alia*, tryptophan and choline chloride; and WO 83/00085, page 10 discloses a composition comprising phenylalanine, threonine and tryptophan administered together with choline.

However all these prior compositions contain other amino acids which would compete with our required amino acid for uptake into the brain.

The invention also provides a composition according to the invention in two-part form, a part A comprising the component (a) and a part B comprising the component (b). Alternatively the components of the composition are present in admixture.

The composition may be packaged and presented for use in relieving the adverse effects of neurological disease or aging in a patient, including clinical instructions to use the composition for that purpose.

The invention also provides use of a composition comprising (a) at least one amino acid selected from phenylalanine, tyrosine, threonine, and tryptophan; and (b) choline, a choline precursor or a mixture of choline and its precursor; for the manufacture of a medicament for relieving the adverse effects of neurological disease or aging in a patient; the composition being substantially free of other amino acids.

The invention also provides use of a composition comprising (a) at least one amino acid selected from phenylalanine, tyrosine, threonine and tryptophan; and (b) choline, a choline precursor or a mixture of choline and its precursor; for the manufacture of a medicament for enhancing release of a neurotransmitter corresponding to the component (a) and for raising the bloodstream choline of a patient to a level from 10 to 50 nanomoles/ml to release a corresponding amount of brain acetylcholine.

There now follows a description of embodiments of the invention. This description, which is illustrative of product and use aspects of the invention, is given by way of example only, and not by way of limitation of the invention.

Choline or a compound that dissociates to form choline is orally administered to a patient prior to or together with an amino acid which is a precursor to a neurotransmitter, in order to increase blood levels of choline and the amino acid, and thereby to increase the levels of acetylcholine and the other neurotransmitter in the brain. The acetylcholine is synthesized from choline and acetyl CoA in a reaction catalyzed by choline acetyltransferase (CAT); the amino acid is converted to the other neurotransmitter by another enzyme. It has been found that the coadministration of choline or a compound that dissociates to form choline, and the amino acid, potentiates the neurological effects of both the amino acid and the choline or choline precursor.

The choline can be administered as choline salts or esters, e.g. the chloride, bitartrate or stearate, or as a compound that dissociates to choline, e.g. sphingomyelin, cytidine-diphospho-choline, an acylglycerophosphocholine, e.g. lecithin, lysolecithin, glycerophosphatidyl choline, or mixtures thereof. By the term acylglycerophosphocholine as used herein is meant a compound of the formula:

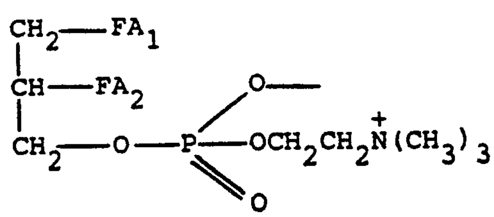

wherein $FA_1$ and $FA_2$ can be the same or different and are fatty acid residues having from 6—26 carbon atoms, usually 16—24 carbon atoms and can be saturated or unsaturated such a palmitic acid, palmitoleic acid, stearic acid, oleic acid, linoleic acid, eicosenoic acid, aracidonic acid, docosahexaenoic acid, eicosapentaenoic acid, linolenic acid, or mixtures thereof. The fatty acid residues of the acylglycerophosphocholine can be varied easily by reacting the acylglycerophosphocholine, e.g. a lecithin with phospholipase Al or A2 (to cleave one fatty acid residue) or then phospholipase B (when desired to cleave both fatty acid residues) and then reacting the cleaved compound with the fatty acid of choice. These choline producing compounds also can be administered to patients having lower than normal plasma choline levels, such as patients experiencing renal dialysis. It is preferred to employ an acylglycerophosphocholine, e.g., lecithin as the choline sources since it is not degraded in the gut in contrast to choline. The choline or compound that dissociates to choline is administered so that a choline level from 10 to 50 nanomoles/ml is attained in the patient's bloodstream; for example the level is at least 20 nanomoles/ml e.g. from 20 to 30 nanomoles/ml.

When administering choline chloride in the form of capsules or tablets, suitable dosages are e.g. from 1 to 30 g/day, preferably 3—20 g/day taken in divided doses 500 to 1000 mg/cap or tab. When choline chloride is administered in liquid form admixed with a conventional liquid carrier such as a sweetened elixir or the like, from 1 to 10 grams/15 ml, preferably from 2 to 5 grams/15 ml can be utilized. When utilizing lecithin in a liquid carrier, it is administered in amounts of e.g. from 0.1 to 50 grams/day. When lecithin is administered in granular form, as a tablet or in a capsule, it is employed in amounts of e.g. from 0.1 to 100 g/day, usually from 30 to 50 g/day. Normally, lecithin is not available as a pure compound and is avilable in admixture with other phospholipids wherein the lecithin comprises about 20—30 weight percent of the mixture.

As indicated above the choline or compound that dissociates to choline is administered concomitantly with the amino acids. The administration of the compositions employed can be effected orally, interperitoneally, subcutaneously, intravenously or intramuscularly.

The amino acids: tyrosine or tyrosine precursor (phenylalanine), threonine or tryptophan, can be used as such, as salts or esters, as peptides or as compounds which are metabolized to give the amino acids *in vitro* (e.g., alpha-keto amino acids). Conveniently, the compositions employed are admixed or dissolved in

any innocuous vehicle such as water or sterile saline solution or in tablet or powder form containing the usual solid diluents or cariers, or as foods or enteral nutrition mixtures. The compositions are administered in concentrations to avoid undesirable side effects. In humans, useful dosages of tyrosine are for example from 0.5 mg/kg to 250 mg/kg (depending on route of administration), preferably from 0.5 mg/kg to 50 mg/kg when given intravenously and from 10 mg/kg to 200 mg/kg when given orally. Threonine and tryptophan doses are similar. The administration of tyrosine or phenylalanine is made in the absence of other amino acids that might compete for uptake in the brain and which themselves do not produce dopamine. When tryptophan is administered, it can be administered with caffeine or another mild stimulant to suppress its effect on sleepiness. Also, the amino acid can be administered with an insulin-releasing carbohydrate e.g., sucrose or glucose in order to lower plasma levels of leucine, isoleucine and valine which would otherwise compete for brain uptake.

## Example

A study was done on rats to show that the concurrent administration of choline with tyrosine produced effects that were much greater than the sum of their individual effects, i.e., that they potentiated each other. Adult male Sprague-Dawley rats were given lesions on one side of the brain; the other side served as a normal control (and had been shown previously to be unaffected by the unilateral lesion). The lesion partially destroyed the dopaminergic neurons running from the substantia nigra to the corpus striatum (i.e., the neurons whose loss is most responsible for Parkinson's Disease in humans, but which also are deficient in normal old people). It has previously been shown that when 75% of the neurons are destroyed, the surviving nigrostriata neurons respond by increasing the frequency with which they fire. Up to a point this suffices, and the animal, or a human with a corresponding loss, does not show symptoms except when stressed; however, beyond a 50—60% lesion, symptoms appear generally. It also has been previously shown that, in such animals, giving supplemental tyrosine had no effect on dopamine synthesis or release (as estimated by measuring the levels of the dopamine metabolites HVA, homovanillic acid, and DOPAC, dihydroxyphenylacetic acid, in the striatum), but did enhance its synthesis and release in the lesioned side.

About a week after placement of the unilateral lesions, animals received either choline chloride (10 nmoles/kg) alone, or tyrosine alone (250 mg/kg), or both, and were killed an hour later; HVA and DOPAC were measured in striata from the unlesioned and lesioned sides (Table). In the unlesioned side, neither tyrosine alone nor choline alone enhanced dopamine release (i.e., as reflected in striatal level of its metabolites HVA and DOPAC). However, giving both compounds did cause significant increases in the metabolites. On the lesioned side, tyrosine alone did, as shown previously, enhance dopamine release (increasing DOPAC from 0.11 to 0.19, and HVA from 0.08 to 0.14); choline alone was without effect. Giving the two precursors caused a vastly greater increase in dopamine release (evaluating DOPAC to 0.28 and HVA to 0.24). The potentiation of tyrosine's effect by choline most likely reflects increased acetylcholine release within the striatum itself; the acetylcholine then acts, trans-synaptically or *via* presynaptic receptors. Since serotoninergic, noradrenergic, and glycinergic neurons appear also to make synaptic contacts with cholinergic neurons, it is apparent that raising blood choline levels will also potentiate the effects of giving their precursors (tryptophan, tyrosine, threonine). Again, the brain will "decide" whether any neurons respond by modulating their firing frequencies.

The ability of tyrosine-plus-choline to enhance dopamine release in the "normal", unlesioned side, provides evidence that mixtures of the precursors will have useful effects in people without overt neurological diseases (e.g., old people who have lost some neurons, but too few to cause overt symptoms; children and young adults with "minimal brain dysfunction").

TABLE

Effect of choline plus tyrosine on dopamine metabolites in rat corpus striatum

| Treatment | Unlesioned Side DOPAC | Unlesioned Side HVA | Lesioned Side DOPAC | Lesioned Side HVA |
|---|---|---|---|---|
| control | 0.52 | 0.48 | 0.11 | 0.08 |
| tyrosine | 0.51 | 0.50 | 0.19* | 0.14* |
| choline | 0.56 | 0.53 | 0.12 | 0.08 |
| tyrosine and choline | 0.66* | 0.58* | 0.28** | 0.24** |

Animals received choline chloride (10 nmoles/kg p.o.), tyrosine (250 mg/kg, i.p.) or both 5—8 days after placement of a partial unilateral nigrostriatal lesion (with 6-hydroxydopamine); they were killed one hour later. Data are given as nanograms/mg tissue.

* $P < 0.05$ differs from corresponding control tissue

** $P < 0.01$ differs from corresponding control tissue

**Claims for the Contracting States: BE CH DE FR GB IT LI LU NL SE**

1. A pharmaceutical composition comprising (a) at least one amino acid selected from phenylalanine, tyrosine, threonine, and tryptophan; and (b) choline, a choline precursor or a mixture of choline and its precursor in amounts sufficient to cause a synergistic enhancement of neurotransmission; the composition being substantially free of other amino acids.

2. A composition according to claim 1, wherein the composition also comprises an excipient.

3. A composition according to claim 1 or claim 2, in pharmaceutical unit dosage form.

4. A composition according to any one of claims 1, 2 and 3, in two-part form, a part A comprising the component (a) and a part B comprising the component (b).

5. A composition according to any one of claims 1, 2 and 3, wherein the components of the composition are present in admixture.

6. Use of a composition comprising (a) at least one amino acid selected from phenylalanine, tyrosine, threonine, and tryptophan; and (b) choline, a choline precursor or a mixture of choline and its precursor; for the manufacture of a medicament for relieving the adverse effects of neurological disease or aging in a patient; the composition being substantially free of other amino acids.

7. Use of a composition comprising (a) at least one amino acid selected from phenylalanine, tyrosine, threonine and tryptophan; and (b) choline, a choline precursor or a mixture of choline and its precursor; for the manufacture of a medicament for enhancing release of a neurotransmitter corresponding to the component (a) and for raising the bloodstream choline of a patient to a level from 10 to 50 nanomoles/ml to release a corresponding amount of brain acetylcholine.

8. Use according to claim 7, wherein the composition is substantially free of other amino acids.

9. A composition of use according to any one of the preceding claims, wherein the choline precursor comprises any of an acylglycerophosphocholine, choline chloride, and cytidine-diphosphocholine.

10. A composition or use according to any one of claims 1 to 8, wherein the choline precursor comprises lecithin.

11. A composition or use according to any one of the preceding claims, wherein the composition also comprises an insulin-releasing carbohydrate.

**Claims for the Contracting State: AT**

1. Use of a composition comprising (a) at least one amino acid selected from phenylalanine, tyrosin, threonine, and tryptophan; and (b) choline, a choline precursor or a mixture of choline and its precursor in amounts sufficient to cause a synergistic enhancement of neurotransmission; for the manufacture of a medicament for relieving the adverse effects of neurological disease or aging in a patient; the composition being substantially free of other amino acids.

2. Use of a composition comprising (a) at least one amino acid selected from phenylalanine, tyrosine, threonine and tryptophan; and (b) choline, a choline precursor or a mixture of choline and its precursor; for the manufacture of a medicament for enhancing release of a neurotransmitter corresponding to the component (a) and for raising the bloodstream choline of a patient to a level from 10 to 50 nanomoles/ml to release a corresponding amount of brain acetylcholine.

3. Use according to claim 1 or claim 2, wherein the choline precursor comprises any of an acylglycerophosphocholine, choline chloride, and cytidinediphosphocholine.

4. Use according to claim 1 or claim 2, wherein the choline precursor comprises lecithin.

5. Use according to any one of the preceding claims, wherein an insulin-releasing carbohydrate is also included in the composition.

**Patentansprüche für die Vertragsstaaten: BE CH DE FR GB IT LI LU NL SE**

1. Pharmazeutische Zusammensetzung, bestehend aus a) zumindest einer aus Phenylalanin, Tyrosin, Threonin und Tryptophan ausgewählten Aminosäure und b) Cholin, einem Cholin-Precursor oder einer Mischung von Cholin und dessen Precursor in zum Hervorrufen einer synergistischen Erhöhung der Neurotransmission ausreichenden Menge, wobei die Zusammensetzung im wesentlichen frei von anderen Aminosäuren ist.

2. Zusammensetzung nach Anspruch 1, bei der die Zusammensetzung ferner eine Trägersubstanz umfaßt.

3. Zusammensetzung nach Anspruch 1 oder Anspruch 2 in Form einer pharmazeutischen Dosierungseinheit.

4. Zusammensetzung nach einem der Ansprüche 1, 2 und 3, in zweiteiliger Form, wobei ein Teil A die Komponente a) und ein Teil B die Komponente b) umfaßt.

5. Zusammensetzung nach einem der Ansprüche 1, 2 und 3, bei der die Komponenten der Zusammensetzung in einer Beimischung vorhanden sind.

6. Verwendung einer Zusammensetzung aus a) zumindest einer aus Phenylalanin, Tyrosin, Threonin und Tryptophan ausgewählten Aminosäure und b) Cholin, einem Cholin-Precursor oder einer Mischung von Cholin und dessen Precursor zur Herstellung eines Medikaments zur Linderung der ungünstigen

Auswirkungen einer neurologischen Krankheit oder des Alterns eines Patienten, wobei die Zusammensetzung im wesentlichen frei von anderen Aminosäuren ist.

7. Verwendung einer Zusammensetzung aus a) zumindest einer aus Phenylalanin, Tyrosin, Threonin und Tryptophan ausgewählten Aminosäure und b) Cholin, einem Cholin-Precursor oder einer Mischung von Cholin und dessen Precursor zur Herstellung eines Medikaments zur Erhöhung der Freisetzung eines Neurotransmitters entsprechend der Komponente a) und zum Anheben des Cholins im Blutstrom eines Patienten auf einen Wert won 10 bis 50 Nanomol/ml zur Freisetzung einer entsprechenden Menge von Acetylcholin im Gehirn.

8. Verwendung nach Anspruch 7, wobei die Zusammensetzung im wesentlichen frei von anderen Aminosäuren ist.

9. Zusammensetzung oder Verwendung nach einem der vorhergehenden Ansprüche, wobei der Cholin-Precursor einen der Stoffe Acylglycerophosphocholin, Cholinchlorid und Cytidin-Diphosphocholin umfaßt.

10. Zusammensetzung oder Verwendung nach einem der Ansprüche 1 bis 8, bei der der Cholin-Precursor aus Lecithin besteht.

11. Zusammensetzung oder Verwendung nach einem der vorhergehenden Ansprüche, wobei die Zusammenzetzung ferner ein Insulin freisetzendes Kohlehydrat umfaßt.

**Patentansprüche für den Vertragsstaat: AT**

1. Verwendung einer Zusammensetzung aus a) zumindest einer aus Phenylalanin, Tyrosin, Threonin und Tryptophan ausgewählten Aminosäure und b) Cholin, einem Cholin-Precursor oder einer Mischung von Cholin und dessen Precursor in zum Hervorrufen einer synergistischen Erhöhung der Neurotransmission ausreichenden Menge zur Herstellung eines Medikaments zur Linderung der ungünstigen Auswirkungen einer neurologischen Krankheit oder des Alterns eines Patienten, wobei die Zusammensetzung im wesentlichen frei von anderen Aminosäuren ist.

2. Verwendung einer Zusammensetzung aus a) zumindest einer aus Phenylalanin, Tyrosin, Threonin und Tryptophan ausgewählten Aminosäure und b) Cholin, einem Cholin-Precursor oder einer Mischung von Cholin und dessen Precursor zur Herstellung eines Medikaments zur Erhöhung der Freisetzung eines Neurotransmitters entsprechend der Komponente a) und zum Anheben des Cholins im Blutstrom eines Patienten auf einen Wert won 10 bis 50 Nanomol/ml zur Freisetzung einer entsprechenden Menge von Acetylcholin im Gehirn.

3. Verwendung nach Anspruch 1 oder 2, wobei der Cholin-Precursor einen der Stoffe Acylglycerophosphocholin, Cholinchlorid und Cytidindiphosphocholin umfäßt.

4. Verwendung nach Anspruch 1 oder 2, wobei der Cholin-Precursor aus Lecithin besteht.

5. Verwendung nach einem der vorhergehenden Ansprüche, wobei ein Insulin freisetzendes Kohlehydrat ebenfalls in der Zusammensetzung enthalten ist.

**Revendications pour les Etats contractants: BE CH DE FR GB IT LI LU NL SE**

1. Composition pharmaceutique comprenant (a) au moins un acide aminé choisi parmi la phénylalanine, la tyrosine, la thréonine et le tryptophanne; et (b) de la choline, un précurseur de la choline ou un mélange de choline et de son précurseur, dans des quantités suffisantes pour produire un renforcment synergique de la neuro-transmission, la composition étant pratiquement exempte d'autres acides aminés.

2. Composition selon la revendication 1, comprenant également un excipient.

3. Composition selon la revendication 1 ou 2, sous forme d'unité posologique pharmaceutique.

4. Composition selon l'une quelconque des revendications 1 à 3, sous la forme d'un produit en deux parties, une partie A qui comprend le composant (a) et une partie B qui comprend le composant (b).

5. Composition selon l'une quelconque des revendications 1 à 3, les composants de la composition étant présents en mélange.

6. Utilisation d'une composition comprenant (a) au moins un acide aminé choisi parmi la phénylalanine, la tyrosine, la thréonine et le tryptophanne; et (b) de la choline, un précurseur de la choline ou un mélange de choline et de son précurseur, pour la préparation d'un médicament destiné à soulager les effets adverses d'affections neurologiques ou du vieillissement chez un patient, la composition étant pratiquement exempte d'autres acides aminés.

7. Utilisation d'une composition comprenant (a) au moins un acide aminé coisi parmi la phénylalanine, la tyrosine, la thréonine et le tryptophanne; et (b) de la choline, un précurseur de la choline ou un mélnge de choline et de son précurseur, pour la préparation d'un médicament destiné à favoriser la libération d'un neurotransmetteur correspondant au composant (a) et à accroître le taux de choline dans le courant sanguin d'un patient jusqu'à un niveau compris entre 10 et 50 nanomoles/ml afin de libérer une quantité correspondante d'acétyl-choline cérébrale.

8. Utilisation selon la revendication 7, la composition étant pratiquement exempte d'autres acides aminés.

9. Composition ou utilisation selon l'une quelconque des revendications 1 à 8, dans laquelle le

précurseur de la choline est constitué par l'une quelconque des substances acylglycérophosphocholine, chlorure de choline et cytidine-diphosphocholine.

10. Composition ou utilisation selon l'une quelconque des revendications 1 à 8, dans laquelle le précurseur de la choline est constitué par la lécithine.

11. Composition ou utilisation selon l'une quelconque des revendications 1 à 10, dans laquelle la composition comprend également un glucide produisant une libération d'insuline.

**Revendications pour l'Etat contractant: AT**

1. Utilisation d'une composition comprenant (a) au moins un acide aminé choisi parmi la phénylalanine, la tyrosine, la thréonine et le tryptophanne; et (b) de la choline, un précurseur de la choline ou un mélange de choline et de son précurseur, dans des quantités suffisantes pour produire un renforcement synergique de la neuro-transmission, pour la préparation d'un médicament destiné à soulager les effets adverses d'affections neurologiques ou du vieillissement chez un patient, la composition étant pratiquement exempte d'autres acides aminés.

2. Utilisation d'une composition comprenant (a) au moins un acide aminé coisi parmi la phénylalanine, la tyrosine, la thréonine et le tryptophanne; et (b) de la choline, un précurseur de la choline ou un mélange de choline et de son précurseur, pour la préparation d'un médicament destiné à favoriser la libération d'un neurotransmetteur correspondant au composant (a) et à accroître le taux de choline dans le courant sanguin d'un patient jusqu'à un niveau compris entre 10 et 50 nanomoles/ml afin de libérer une quantité correspondante d'acétyl-choline cérébrale.

3. Utilisation selon la revendication 1 ou 2, dans laquelle le précurseur de la choline est constitue par l'une quelconque des substances acylglycérophosphocholine, chlorure de choline et cytidine-diphosphocholine.

4. Utilisation selon la revendication 1 ou 2, dans laquelle le précurseur de la choline est constitué par la lécithine.

5. Utilisation selon l'une quelconque des revendications 1 à 4, dans laquelle un glucide produisant une libération d'insuline est également contenu dans la composition.